# EUROPÄISCHE PATENTSCHRIFT

(11) **EP 2 680 910 B1**
(45) Veröffentlichungstag und Bekanntmachung des Hinweises auf die Patenterteilung: **27.04.2022**
(21) Anmeldenummer: 12706035.8
(22) Anmeldetag: 27.02.2012
(51) Int. Cl.: A61M 5/315, A61M 5/24

(54) **VORRICHTUNG ZUR BETÄTIGUNG EINER KARPULE**
DEVICE FOR ACTUATING A CARPULE
DISPOSITIF D'ACTIONNEMENT D'UNE CARPULE

(30) Priorität: 01.03.2011 EP 11001664; 12.05.2011 EP 11003945
(43) Veröffentlichungstag der Anmeldung: 08.01.2014
(73) Patentinhaber: Boehringer Ingelheim Microparts GmbH, 44227 Dortmund (DE)
(72) Erfinder: JOEDICKE, Thorsten, 55216 Ingelheim am Rhein (DE); LANGE, Berthold, 55216 Ingelheim am Rhein (DE); WILLMS, Thomas, 55216 Ingelheim am Rhein (DE); YU, Ying, 55216 Ingelheim am Rhein (DE)
(74) Vertreter: Simon, Elke Anna Maria
(86) Internationale Anmeldenummer: PCT/EP2012/053249
(87) Internationale Veröffentlichungsnummer: WO 2012/116948

(56) Entgegenhaltungen:
- EP-A1- 0 328 699
- WO-A2-2004/078226
- DE-A1- 10 340 585
- DE-A1-102005 063 311

## Beschreibung

Die vorliegende Erfindung betrifft eine Vorrichtung zur Aufnahme und Betätigung einer Karpule gemäß dem Oberbegriff des Anspruchs 1.

Bei einer Karpule im Sinne der vorliegenden Erfindung handelt es sich insbesondere um eine Ampulle mit einer Arzneimittelformulierung und einem darin verschiebbaren Kolben. In einem weiteren Sinne kann sich bei der Karpule vorzugsweise auch um eine Spritze oder einen sonstigen Arzneimittelbehälter handeln.

Unter dem Begriff "Arzneimittelformulierung" und "Arzneimittel" sind bei der vorliegenden Erfindung über Medikamente hinaus auch Therapeutika, Diagnostika oder dergleichen, insbesondere also jede Art von Mitteln zur Injektion zu verstehen.

Das Arzneimittel bzw. die Arzneimittelformulierung kann in der Karpule bereits in flüssiger Form vorliegen. In diesem Fall wird die flüssige Arzneimittelformulierung mittels des Kolbens beim Betätigen der Karpule, insbesondere durch Verschieben des Kolbens, über einen Auslass ausgetragen.

Die WO 2004/078226 A2 zeigt einen Antriebsmechanismus für eine Abgabevorrichtung beispielsweise in einem als sogenannten Pen ausgebildeten Injektor, der eine Dosiereinstellungsmöglichkeit aufweist und mit dem die Verabreichung von einem medizinischen Produkt aus einer Mehrdosis-Kartusche möglich ist. Die das medizinische Produkt beinhaltende Kartusche weist gegenüber ihrer Ausgabeseite, die mit einer Nadeleinheit verbunden werden kann, einen verschiebbaren Kolben auf, wobei das medizinische Produkt durch Verschiebung des Kolbens in Richtung Ausgabeseite ausgebracht wird. Der Antriebsmechanismus weist eine Dosiswählhülse mit einem schraubenförmigen Gewinde mit einer ersten Steigung und eine zweiteilige Kolbenstange zur Verschiebung des Kolbens auf. Die zweiteilige Kolbenstange weist einen äußeren Teil mit einem schraubenförmigen Außengewinde mit einer zweiten Steigung auf, wobei die erste Steigung des Gewindes der Dosiswählhülse gleich der Summe der zweiten Steigung des Gewindes des äußeren Teils der Kolbenstange und einer dritten Steigung eines Gewindes an einem inneren Teil der Kolbenstange ist.

Die DE 102005063311 A1 zeigt eine ebenfalls eine auf dem Kolbenstangenprinzip basierenden Verabreichungsvorrichtung mit einer zweiteiligen Fördereinrichtung. Die Verabreichungsvorrichtung weist eine Anzeigetrommel als Teil einer Dosiereinrichtung auf, mittels der eine Produktdosis einstellbar ist. Die Vorrichtung umfasst ein Gehäuse mit einer Aufnahme für ein zu verabreichendes fluides Produkt, ein erstes, relativ zum Gehäuse bewegbares Förderglied und ein zweites Förderglied, das mit dem ersten in einem Gewindeeingriff steht und gegenüber diesem rotatorisch und gegen die Förderrichtrung translatorisch bewegbar ist. Die Dosiereinrichtung ist mit einem zweiten Gewindeeingriff relativ zum Gehäuse rotatorisch und in und gegen die Förderrichtung translatorisch bewegbar, wobei eine Kupplung das zweite Förderglied und die Anzeigetrommel in einem Kupplungseingriff verdehgesichert miteinander verbindet, wobei dieser Kupplungseingriff durch Betätigung der Vorrichtung lösbar ist.

Die Arzneimittelformulierung bzw. ein Arzneimittel oder Wirkstoff kann jedoch auch in trockener Form in der Karpule vorliegen und vor dem Applizieren bzw. Austragen zunächst mit einem Lösungsmittel bzw. einer Flüssigkeit gemischt werden. in diesem Fall handelt es sich insbesondere um eine Mehrkammerkarpule, wie beispielsweise aus der DE 103 40 585 A1 oder WO 2008/148518 A1 bekannt. Die Flüssigkeit bzw. das Lösungsmittel ist in einer Kammer der Karpule aufgenommen und wird bei Verschieben des Kolbens in eine andere Kammer mit dem Arzneimittel bzw. Wirkstoff überführt und damit gemischt. So wird die flüssige Arzneimittelformulierung in einem Mischvorgang oder Mischschritt - hier kurz als "Mischen" bezeichnet - in der Karpule gebildet und anschließend bei weiterem Verschieben des Kolbens ausgetragen bzw. appliziert.

Die bekannten Vorrichtung zur Aufnahme und Betätigung einer Karpule weisen jeweils einen Stößel zur Betätigung bzw. Verschiebung des Kolbens der Karpule auf. Die bekannten Vorrichtungen sind jedoch hinsichtlich Aufbau und Handhabung nicht optimal.

Der vorliegenden Erfindung liegt die Aufgabe zugrunde, eine Vorrichtung anzugeben, wobei ein einfacher, robuster und/oder kompakter Aufbau zur vorzugsweise vollständigen Aufnahme der Karpule und/oder eine sehr einfache und/oder sichere Bedienung ermöglicht oder erleichtert wird bzw. werden.

Die obige Aufgabe wird durch eine Vorrichtung gemäß Anspruch 1 gelöst. Vorteilhafte Weiterbildungen sind Gegenstand der Unteransprüche.

Hierin beschrieben ist ein Stößel, der die Karpule betätigend bzw. einen Kolben der Karpule verschiebend, durch Verdrehen teleskopisch verlängert. Der Stößel ist hierzu mehrteilig ausgebildet. Dies ist einer einfachen Handhabung zuträglich, da der mit der axialen Verschiebung des Kolbens üblicherweise einhergehende axiale Hub eines Bedienelements entsprechend der teleskopischen Verlängerung minimiert oder reduziert werden kann. Weiter wird ein einfacher, robuster und/oder kompakter Aufbau ermöglicht.

Ein weiterer Aspekt der vorliegenden Erfindung liegt darin, dass mehrere unterschiedliche Bedienbewegungen, vorzugsweise in unterschiedliche Richtungen erfolgende Bewegungen, insbesondere mindestens eine Drehbewegung und eine Axialbewegung, kombiniert werden oder erforderlich sind, um die Vorrichtung zu bedienen bzw. die Karpule zu betätigen bzw. den Kolben zu verschieben. Dies gestattet insbesondere bei mehrstufigen Verfahrensabläufen, besonders bevorzugt mit einem Mischvorgang in der Karpule, eine einfache, intuitive und/oder sichere Bedienung. Insbesondere werden verschiedene Vorgänge, wie Öffnen bzw. Anstechen der Karpule, Mischen, Primen (Austreiben von Luft) und Austragen der flüssigen Arzneimittelformulierung, durch unterschiedliche Bedienbewegungen realisiert oder bewirkt.

Hierin beschrieben ist ein Bedienelement der Vorrichtung nacheinander in entgegengesetzte Richtungen drehbar, um die Karpule zu betätigen und/oder um unterschiedliche Betätigungen bzw. Vorgänge in der Karpule zu bewirken.

Vorzugsweise wird eine trockene Arzneimittelformulierung zunächst von bzw. in einem flüssigen Lösungsmittel in der Karpule gelöst und dann das Lösungsmittel mit der gelösten Arzneimittelformulierung ausgegeben bzw. ausgetragen. So werden einerseits die Vorteile einer trockenen Arzneimittelformulierung, beispielsweise die gute Lagerstabilität, und andererseits die Vorteile einer flüssigen Arzneimittelformulierung bzw. flüssigen Arzneimittelzuführung, realisierbar. Beispielsweise ist auch ein Mischen zweier Flüssigkeiten und/oder flüssiger Arzneimittelformulierungen in der Karpule vor dem Austrag möglich.

Weitere Aspekte, Merkmale, Eigenschaften und Vorteile der vorliegenden Erfindung ergeben sich aus den Ansprüchen und aus der folgenden Beschreibung einer bevorzugten Ausführungsform anhand der Zeichnung. Es zeigt:
- Fig. 1: eine schematische Darstellung einer vorschlagsgemäßen Vorrichtung im vormontierten Zustand mit eingesetzter Karpule;
- Fig. 2: einen schematischen Schnitt der Vorrichtung gemäß Fig. 1;
- Fig. 3: eine perspektivische Darstellung eines Vorderteils der Vorrichtung;
- Fig. 4: eine perspektivische Ansicht eines Kopfteils mit aufgesetzter Abdeckung der Vorrichtung;
- Fig. 5: eine perspektivische Ansicht eines Mittelteils der Vorrichtung;
- Fig. 6: eine perspektivische Ansicht eines Einsatzteils der Vorrichtung;
- Fig. 7: eine perspektivische Ansicht eines Hinterteils der Vorrichtung;
- Fig. 8: eine perspektivische Ansicht eines Innenteils der Vorrichtung;
- Fig. 9: eine perspektivische Ansicht eines Betätigungsteils der Vorrichtung;
- Fig. 10: eine schematische, ausschnittsweise Darstellung einer Verrastung des Vorderteils mit dem Mittelteil im vormontierten Zustand;
- Fig. 11: einen schematischen Schnitt der Vorrichtung bei geöffneter Karpule;
- Fig. 12: eine ausschnittsweise Vergrößerung von Fig. 11 im Bereich des Kopfteils bzw. vorderen Endes;
- Fig. 13: eine ausschnittsweise, schematische Darstellung einer Verrastung des Vorderteils mit dem Innenteil;
- Fig. 14: einen schematischen Schnitt der Vorrichtung gemäß Fig. 2 entlang Linie XIV-XIV;
- Fig. 15: einen schematischen Schnitt der Vorrichtung nach Mischen von Flüssigkeit und Arzneimittel in der Karpule;
- Fig. 16: einen vergrößerten Ausschnitt von Fig. 15 im Bereich des Übergangs des Hinterteils zum Betätigungsteil;
- Fig. 17: eine schematische, ausschnittsweise Darstellung einer Drehblockierung des Hinterteils durch das Einsatzteil und Innenteil;
- Fig. 18: einen schematischen Schnitt der Vorrichtung nach dem Primen; und
- Fig. 19: einen schematischen Schnitt der Vorrichtung nach dem Austragen bzw. Ausgeben der Arzneimittelformulierung.

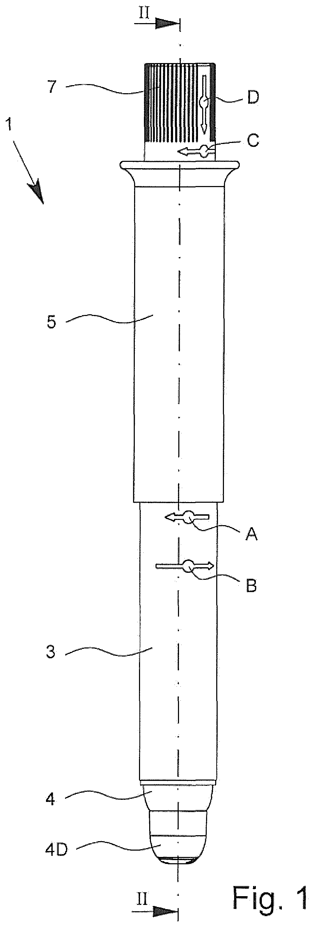

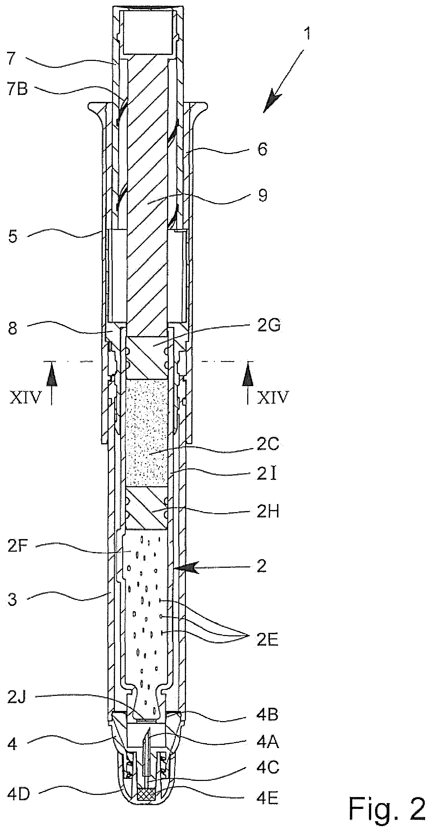

Eine vorschlagsgemäße Vorrichtung 1 ist in Fig. 1 in einer schematischen Seitenansicht und in Fig. 2 in einem schematischen Schnitt gezeigt. Die Vorrichtung 1 wird in einem vormontierten bzw. bereits zusammengebauten Zustand gezeigt, wobei eine Karpule 2, insbesondere im eingangs genannten Sinne oder ein sonstiger Arzneimittelbehälter, bereits in die Vorrichtung 1 aufgenommen oder eingesetzt ist, wie aus dem Schnitt gemäß Fig. 2 ersichtlich.

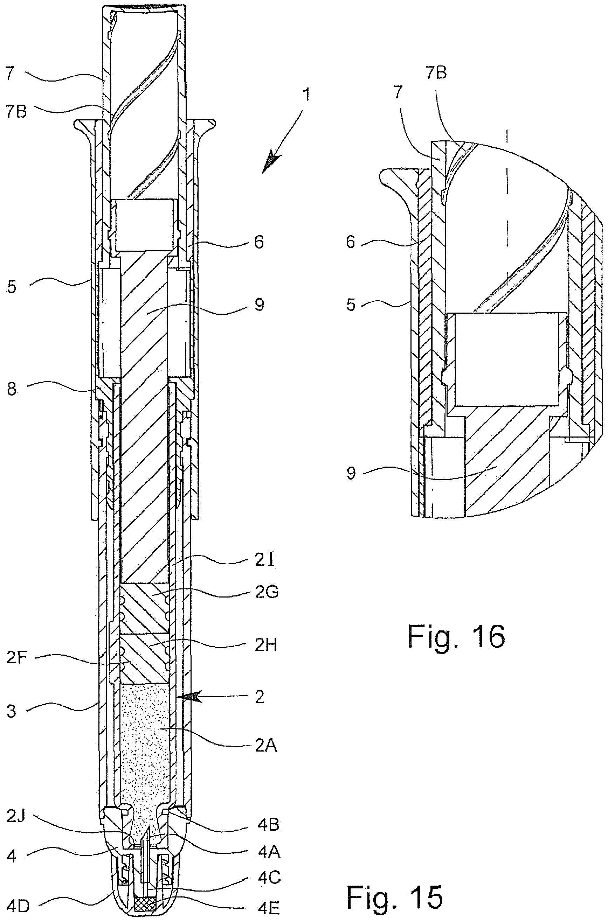

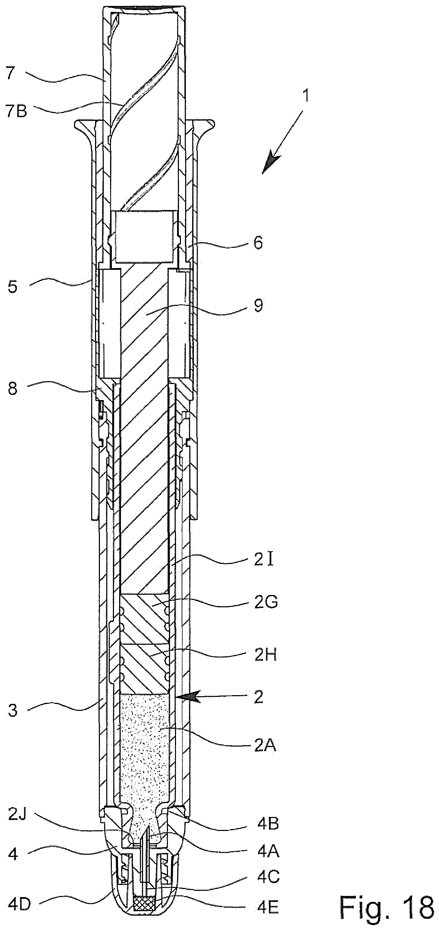

Die Karpule 2 dient der Bereitstellung einer flüssigen Arzneimittelformulierung 2A, wie in Fig. 15 und 18 angedeutet, die mittels der Vorrichtung 1 ausgebbar bzw. austragbar ist, und zwar insbesondere durch entsprechende Bedienung der Vorrichtung 1 und daraus resultierende (vorzugsweise manuelle) Betätigung der Karpule 2.

Beim Darstellungsbeispiel ist die Karpule 2 vorzugsweise als Mehrkammerkarpule ausgebildet. Sie weist eine erste Kammer 2B mit einem Lösungsmittel oder einer Flüssigkeit 2C sowie eine zweite Kammer 2D mit einem von der Flüssigkeit 2G getrennten, insbesondere trockenen oder ggf. auch flüssigen Wirkstoff oder Arzneimittel 2E auf, wie in Fig. 2 angedeutet. Die Flüssigkeit 2C kann in einem Mischvorgang oder Überführungsschritt (kurz als "Mischen" bezeichnet) vorzugsweise mittels eines Überströmkanals 2F von der ersten Kammer 2B in die zweite Kammer 2D strömen und das Arzneimittel 2E lösen, um die flüssige Arzneimittelformulierung 2A zunächst in der Karpule 2 zu bilden. Anschließend kann diese Arzneimittelformulierung 2A dann ausgegeben bzw. ausgetragen werden.

Die Karpule 2 weist vorzugsweise einen ersten Kolben 2G und besonders bevorzugt einen zweiten Kolben 2H auf. Im Auslieferungszustand bzw. im unbenutzten Zustand wird die Flüssigkeit 2C in der ersten Kammer 2B, insbesondere zwischen den beiden Kolben 2G und 2H, gehalten. Jedoch sind auch andere Realisierungen möglich, beispielsweise kann anstelle des Überströmkanals 2F und/oder zweiten Kolbens 2H beispielsweise auch eine Membran- oder Ventilanordnung oder dergleichen vorgesehen werden.

Bei dem vorzugsweise trockenen oder getrockneten Arzneimittel 2E kann es sich beispielsweise um ein Lyophilisat mit einem entsprechenden Wirkstoff oder dergleichen handeln. Das trockene Arzneimittel 2E liegt vorzugsweise in eingetrockneter Form und/oder in Pulverform, Pelletform oder dergleichen vor.

Beim Darstellungsbeispiel wird die flüssige Arzneimittelformulierung 2A erst bei Benutzung bzw. Betätigung der Karpule 2 gemischt bzw. bereitgestellt. Jedoch kann die Karpule 2 grundsätzlich die flüssige Arzneimittelformulierung 2A auch bereits vorgemischt bzw. bereits in flüssiger Form enthalten. Beispielsweise kann die Karpule 2 auch als Einkammerkarpule ausgeführt sein. Die vorschlagsgemäße Vorrichtung 1 ist vorzugsweise auch hierfür einsetzbar, so dass die nachfolgenden Erläuterungen dann insbesondere entsprechend gelten.

Die Karpule 2 weist ein vorzugsweise im Wesentlichen hohlzylindrisches und/oder längliches Gehäuse 2I auf.

Die Kolben 2G, 2H sind in der Karpule 2 bzw. in deren Gehäuse 2I vorzugsweise verschieblich geführt. Insbesondere ist die Karpule 2 an einer Betätigungsseite, bei der Darstellung gemäß Fig. 2 am oberen Ende, offen oder öffenbar ausgebildet, so dass eine Betätigung der Karpule 2 durch Einwirken auf den ersten Kolben 2G, besonders bevorzugt ein Drücken oder Verschieben des Kolbens 2G, möglich ist.

Die Karpule 2 ist auslassseitig, insbesondere an einem dem Betätigungsende gegenüberliegenden Ende, hier dem vorderen oder unteren Ende, mit einem öffenbaren Verschluss 2J, wie einem Septum, einer Membran oder dergleichen, im Auslieferungszustand und in dem in Fig. 1 und 2 dargestellten, eingebauten Zustand (zunächst) verschlossen.

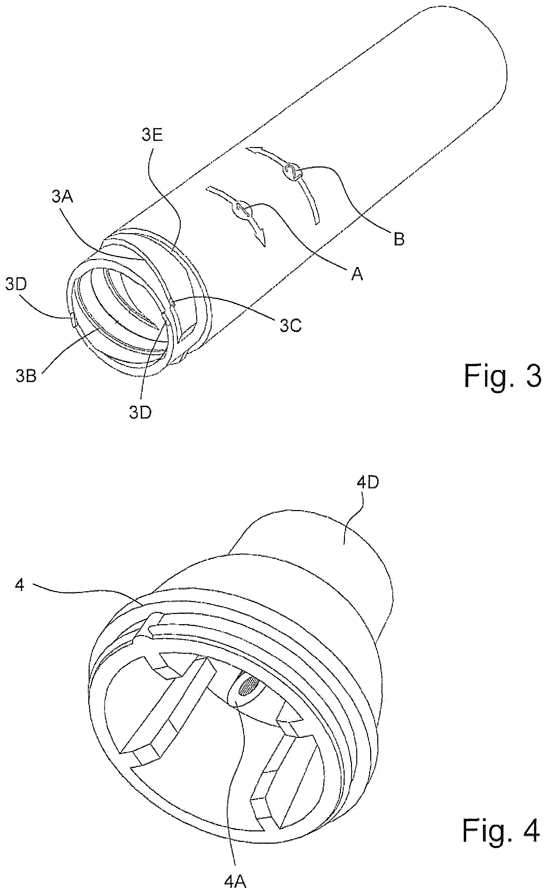

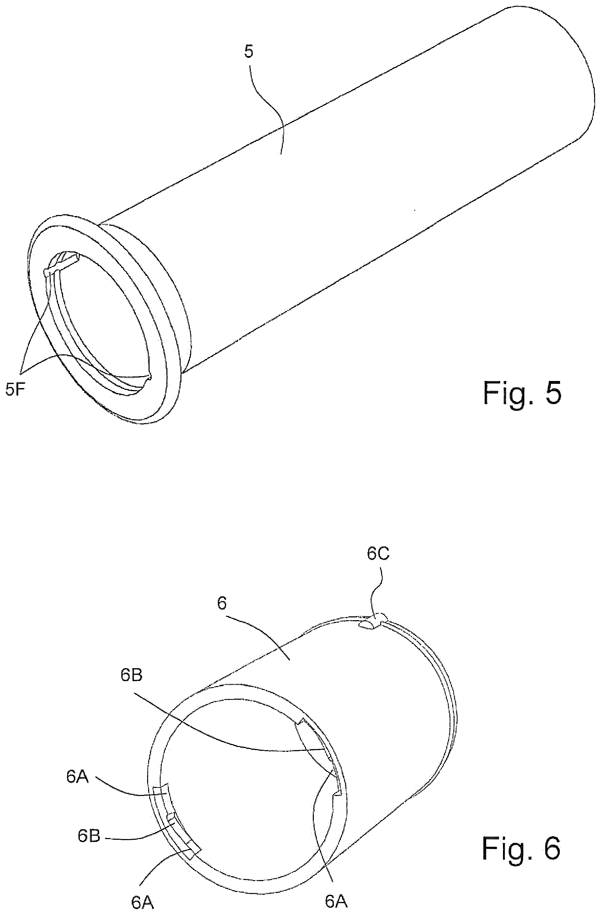

Die Vorrichtung 1 weist beim Darstellungsbeispiel vorzugsweise ein Vorderteil 3, ein Kopfteil 4, ein Mittelteil 5, ein Einsatzsteil 6, ein Hinterteil 7, ein Innenteil 8 und/oder ein Betätigungsteil 9 auf, wie insbesondere in den perspektivischen Darstellungen gemäß Fig. 3 bis 9 gezeigt.

Die Vorrichtung 1 ist vorzugsweise zum Öffnen bzw. Anstechen der Karpule 2 bzw. des Verschlusses 2J ausgebildet. Hierzu weist die Vorrichtung 1 bzw. deren Kopfteil 4 vorzugsweise ein Öffnungs- oder Anstechelement 4A, beispielsweise in Form einer nach innen weisenden Kanüle 4A oder dergleichen, auf, wie in Fig. 2 angedeutet. Das Anstechelement 4A ist beim Darstellungsbeispiel vorzugsweise von dem Kopfteil 4 gehalten bzw. in dieses eingegossen.

Um ein unerwünschtes Öffnen bzw. Anstechen der Karpule 2 im vormontierten bzw. eingebauten Zustand zu vermeiden, ist die Vorrichtung 1 vorzugsweise derart ausgebildet, dass die Karpule 2 form- und/oder kraftschlüssig mit dem Verschluss 2J beabstandet zu dem Anstechelement 4A gehalten wird. Beim Darstellungsbeispiel weist die Vorrichtung 1 bzw. deren Kopfteil 4 einen ggf. etwas konisch ausgebildeten Ringabschnitt 4D auf, der die Karpule 2 insbesondere außenseitig bzw. am unteren Ende bzw. Auslassende reibschlüssig derart beabstandet zum Anstechelement 4A hält, dass der Verschluss 2J nicht versehentlich bzw. vorzeitig geöffnet oder angestochen wird.

Beim Darstellungsbeispiel weist die Vorrichtung 1 bzw. deren Kopfteil 4 einen Auslass 4C auf, der sich vorzugsweise fluidisch an die Kanüle anschließt und/oder einer Ausgabe der flüssigen Arzneimittelformulierung 2A dient. Der Auslass 4C ist vorzugsweise als Standardanschluss, beispielsweise zum Anschluss an einen nicht dargestellten Katheter oder dergleichen, ausgebildet.

Die Vorrichtung 1 bzw. deren Kopfteil 4 bzw. der Auslass 4C ist vorzugsweise zunächst durch eine abnehmbare Abdeckung 4D und/oder einen Saugkörper 4E abgedeckt. Der Saugkörper 4E ist vorzugsweise schwammartig ausgebildet und/oder von der Abdeckung 4D gehalten oder aufgenommen. Die Abdeckung 4D ist ihrerseits vorzugsweise rastend oder klemmend auf das Kopfteil 4 bzw. die Vorrichtung 1 aufgesetzt. Jedoch kann diese auch aufgeschraubt oder in sonstiger Weise verbunden sein.

Das Kopfteil 4 ist vorzugsweise rastend, klemmend und/oder unlösbar mit dem Vorderteil 3 der Vorrichtung 1 verbunden.

Das Kopfteil 4 ist vorzugsweise deckel- oder kappenartig ausgebildet.

Das Vorderteil 3 der Vorrichtung 1 ist vorzugsweise zumindest im Wesentlichen hülsenartig und/oder hohlzylindrisch ausgebildet.

Das Vorderteil 3 bildet vorzugsweise ein erstes Bedienelement der Vorrichtung 1. Hierauf wird später noch näher eingegangen.

Das Vorderteil 3 ist mit dem Mittelteil 5 verbindbar und bildet mit diesem Zusammen vorzugsweise ein zumindest im Wesentlichen längliches oder zylindrisches Gehäuse der Vorrichtung 1.

Das Gehäuseteil 5 ist insbesondere zumindest im Wesentlichen hülsenartig oder hohlzylindrisch ausgebildet.

Im vormontierten Zustand ist das Gehäuseteil 3 bereits mit dem Mittelteil 5 verbunden, insbesondere durch entsprechendes Verschrauben. Beim Darstellungsbeispiel weist das Vorderteil 3 an seinem dem Mittelteil 5 zugewandten Ende vorzugsweise ein Außengewinde 3A zum direkten Verbinden oder Verschrauben mit dem Mittelteil 5 auf. Insbesondere kann das Mittelteil 5 über entsprechende innenseitige Vorsprünge oder ein Innengewinde 5A, wie in Fig. 2 nur schematisch angedeutet, mit dem Außengewinde 3A in Eingriff treten bzw. verbunden werden.

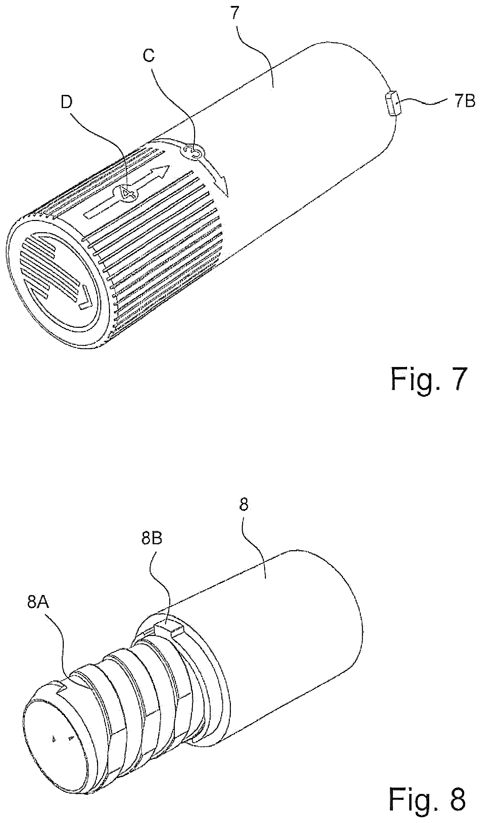

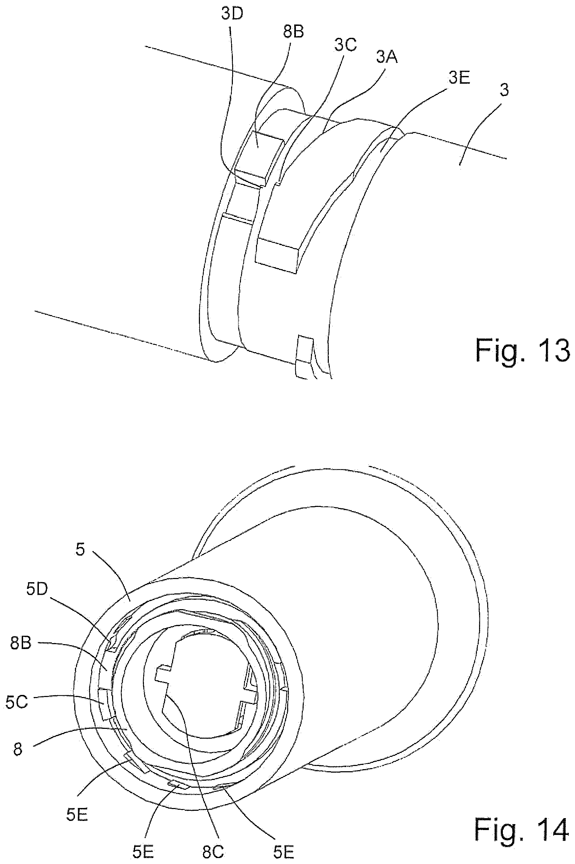

Alternativ oder zusätzlich kann das Vorderteil 3 insbesondere über das Innengewinde 3B mit dem Innenteil 8 verbunden, insbesondere verschraubt, sein oder werden, wobei das Innenteil 8 vorzugsweise seinerseits im und/oder am Mittelteil 5 (axial) widergelagert bzw. (drehbar) gelagert ist, besonders bevorzugt nur in eine Richtung drehbar, wie in Fig. 14 gezeigt, worauf später noch näher eingegangen wird. Das Innenteil 8 weist daher vorzugsweise ein entsprechendes Außengewinde (A, wie insbesondere in Fig. 8 angedeutet, zur Verbindung mit dem Innengewinde 3B des Vorderteils 3 auf.

Zur Montage der Vorrichtung 1 werden vorzugsweise zunächst zwei Baugruppen bzw. Baueinheiten gebildet, und zwar eine erste Baueinheit bestehend aus dem Vorderteil 3 und Kopfteil 4 und eine zweite Baueinheit bestehend aus den weiteren Teilen 5 bis 9.

Das Einsatzteil 6 ist vorzugsweise zumindest im Wesentlichen hülsenartig oder hohlzylindrisch ausgebildet.

Das Einsatzteil 6 weist vorzugsweise wenigstens einen radialen Vorsprung 6C, beim Darstellungsbeispiel zwei radiale Vorsprünge 6C, zur drehfesten Verbindung mit dem Gehäuseteil 5 auf. Insbesondere greifen die Vorsprünge 6C in entsprechende axial offene Ausnehmungen 5F des Mittelteils 5 beim axialen Einsetzen bzw. Einrasten des Einsatzteils 6 in das Mittelteil 5.

Vor dem Einsetzen des Einsatzteils 6 in das Mittelteil 5 wird vorzugsweise das Hinterteil 7 von unten in das Einsatzteil 6 eingesetzt, so dass die Vorsprünge 7B in den Ausnehmungen 6A zu liegen kommen. Des Weiteren wird vorher das Innenteil 8 vom rückwärtigen bzw. hinterteilseitigen Ende des Mittelteils 5 aus in das Mittelteil 5 eingesetzt. Anschließend kann das Einsatzteil 6 zusammen mit dem in das Hinterteil 7 eingeschraubten Betätigungsteil 9 in das Mittelteil 5 eingebaut bzw. eingesetzt werden. So ergibt sich ein einfacher Zusammenbau.

Nach der Vormontage der beiden genannten Baueinheiten wird die Karpule 2 eingesetzt, insbesondere zunächst in das Mittelteil 5 bzw. Innenteil 8. Anschließend werden die beiden Baueinheiten miteinander verbunden, insbesondere verschraubt. Besonders bevorzugt wird das Vorderteil 3 auf die Karpule 2 aufgeschoben und mit dem Mittelteil 5 und/oder Innenteil 8 verschraubt, bis der vormontierte Zustand erreicht wird.

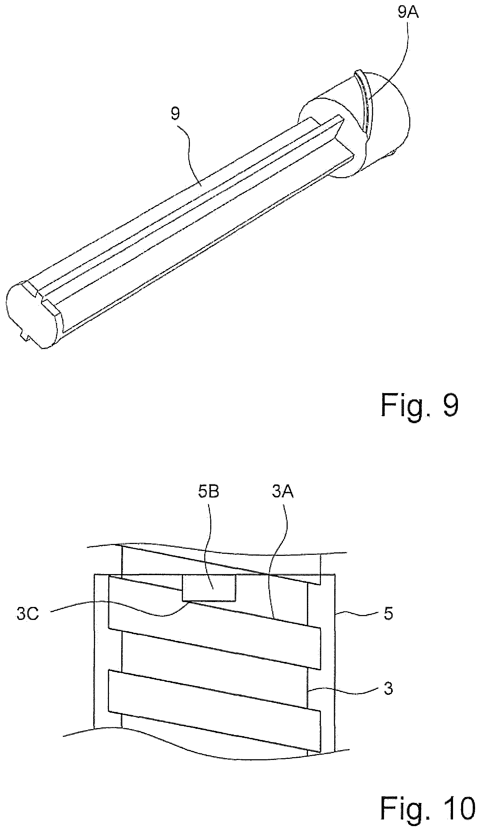

Zum Verschrauben wird das Gehäuseteil 3 vorzugsweise in Richtung des Pfeils A, wie in Fig. 1 dargestellt, relativ zu dem Mittelteil 5 gedreht. Hierbei erfolgt jedoch vorzugsweise kein vollständiges Verschrauben bzw. Einschrauben des Gewindeteils 3, sondern nur ein Verschrauben bis zum Erreichen der vormontierten Position (einer Zwischenposition). Diese vormontierte Position wird insbesondere durch eine (erste) Rastung angezeigt, wie beispielhaft anhand von Fig. 10 gezeigt. Ein innenseitiger Vorsprung 5B am Mittelteil 5 greift hier in eine Rastausnehmung 3C am Vorderteil 3, insbesondere an einem Gewindegang des Außengewindes 3A, an oder ein. Die Rastausnehmung 3C bzw. die Verrastung ist vorzugsweise derart ausgebildet, dass das Erreichen dieser Rastposition bzw. der vormontierten Position durch ein "Klicken" oder sonstiges Geräusch angezeigt wird und/oder dass ein Zurückdrehen nicht mehr möglich ist, sondern nach Überwinden einer entsprechenden Kraft, beispielsweise unter entsprechender Verformung, nur ein Weiterdrehen in Richtung A, also nur ein weiteres Zusammenschrauben des Vorderteils 3 mit dem Mittelteil 5 und/oder Innenteil 8 möglich ist.

Ausgehend von dem vormontierten bzw. eingebauten Zustand untergliedert sich die Betätigung bzw. Benutzung der Karpule 2 und insbesondere auch die Bedienung der Vorrichtung 1 vorzugsweise in vier Abschnitte, Schritte bzw. Vorgänge, und zwar in: erstens das Öffnen (Anstechen der Karpule 2 bzw. des Verschlusses 2J); zweitens das Mischen (Überführen der Flüssigkeit 2C aus der ersten Kammer 2B in die zweite Kammer 2D bzw. zum Arzneimittel 2E); drittens das Primen (Verdrängen von Luft aus der Karpule 2, dem Kopfteil 4, der Kanüle 4A und/oder dem Auslass 4C); und viertens das Austragen (Ausgeben der flüssigen Arzneimittelformulierung 2A durch den Auslass 4C bzw. über das Kopfteil 4 aus der Vorrichtung 1).

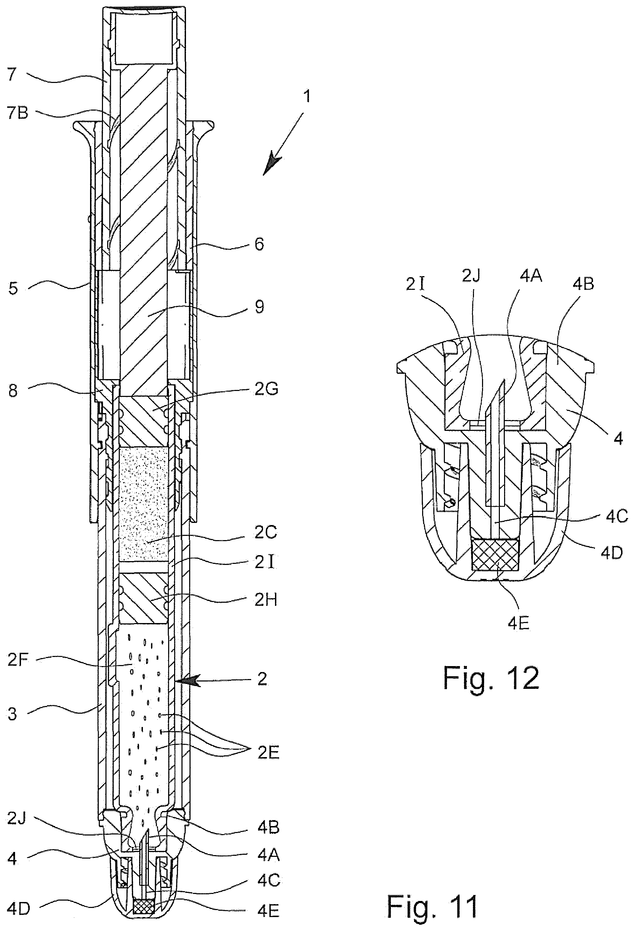

Zum Öffnen erfolgt eine erste Bedienung der Vorrichtung 1 bzw. eine Bedienbewegung in Richtung A, also wie durch Pfeil A in Fig. 1 angedeutet. Insbesondere erfolgt ein Drehen bzw. Einschrauben des ersten Bedienelements, hier des Vorderteils 3, besonders bevorzugt relativ zu dem Mittelteil 5 und/oder Innenteil 8. Durch diese Bedienung wird das Kopfteil 4 mit dem Öffnungs- bzw. Anstechelement 4A axial zu der Karpule 2 bewegt, so dass der Verschluss 2J der Karpule 2 geöffnet, insbesondere angestochen wird. Dieser geöffnete bzw. angestochene Zustand ist in dem schematischen Schnitt gemäß Fig. 11 und der ausschnittsweisen Vergrößerung gemäß Fig. 12 gezeigt.

Bei der axialen Bewegung des Kopfteils 4 bzw. Anstechelements 4A zur Karpule 2 hin stützt sich die Karpule 2 bzw. deren Gehäuse 2E vorzugsweise am anderen Ende am Innenteil 8 oder dergleichen ab, so dass der Reibschluss zwischen dem Ringabschnitt 4B und dem auslassseitigen Ende der Karpule 2 überwunden wird und die Karpule 2 mit ihrem auslassseitigen Ende in das Kopfteil 4 hineingeschoben wird bzw. umgekehrt das Kopfteil 4 auf die Karpule 2 geschoben wird.

Es ist anzumerken, dass sowohl im vormontierten Zustand, wie in Fig. 1 und 2 dargestellt, als auch in geöffneten Zustand, wie in Fig. 11 und 12 dargestellt, der erste Kolben 2G der Karpule 2 noch nicht betätigt, also noch nicht eingeschoben bzw. verschoben wurde.

Es ist anzumerken, dass ausgehend von dem vormontierten Zustand zunächst eine etwas erhöhte Kraft beim Drehen des ersten Bedienelements bzw. Vorderteils 3 aufgewendet werden muss, um die Rastung durch Eingriff des Vorsprungs 5B in die Rastausnehmung 3C zu überwinden, wie bereits angedeutet.

Es ist auch möglich, dass weitere Rastausnehmungen 3C oder dergleichen entlang des Gewindegangs des Außengewindes 3A vorgesehen bzw. angeordnet sind, um eine Art Ratsche zu bilden, so dass ein unerwünschtes Rückdrehen in die entgegengesetzte Richtung nicht mehr möglich ist. Jedoch sind hier auch andere konstruktive Lösungen möglich.

Wenn die Bewegung in Richtung A, also die Bedienung zum Öffnen, abgeschlossen ist, verrastet das erste Bedienelement bzw. Vorderteil 3 vorzugsweise derart mit dem Innenteil 8, wie insbesondere in Fig. 13 schematisch angedeutet, dass das Vorderteil 3 in entgegengesetzter Drehrichtung gemäß Pfeil B (siehe Fig. 1) mit dem Innenteil 8 drehfest gekoppelt ist oder wird. Mit anderen Worten, wenn das erste Bedienelement bzw. Vorderteil 3 der Vorrichtung 1 in entgegengesetzter Richtung bzw. in Richtung B gedreht wird (zu A entgegengesetzte Bedienbewegung B), wird nun das Innenteil 8 mitgedreht.

Für die vorgenannte Verrastung bzw. Drehkopplung weist das Vorderteil 3 beim Darstellungsbeispiel vorzugsweise einen axialen Hinterschnitt, Anschlag oder Vorsprung 3D auf, der nach Vorbeibewegen an einem radialen Vorsprung 8B des Innenteils 8 (das Vorbeibewegen ist durch elastische Materialverformung möglich) zur Anlage kommt, um in die entgegengesetzte Richtung die gewünschte Drehkopplung zu erreichen.

Bei Beendigung der Bewegung in Richtung A bzw. des Einschraubens des Vorderteils 3 ist das Vorderteil 3 vorzugsweise vollständig auf das Innenteil 8 aufgeschraubt bzw. in die Vorrichtung 1 bzw. das Mittelteil 5 eingeschraubt.

Das Außengewinde 3A des Vorderteils 3 ist vorzugsweise derart ausgebildet, dass im vollständig eingeschraubten Zustand das Vorderteil 3 im Mittelteil 5 frei drehbar ist, also ohne dass dieses axial wieder herausgeschraubt wird. Hierzu ist das Vorderteil 3 bzw. Außengewinde 3A insbesondere mit einem entsprechenden Freigang 3E versehen, wie in Fig. 3 angedeutet.

Das Innenteil 8 ist in der Vorrichtung 1 bzw. dem Gehäuseteil 5 vorzugsweise derart aufgenommen, dass es nur in einer Richtung, beim Darstellungsbeispiel nur in der dem Öffnen entgegengesetzten Richtung bzw. in Richtung B bewegbar oder drehbar ist, wie insbesondere aus der Darstellung gemäß Fig. 14 ersichtlich. Fig. 14 stellt einen schematischen Schnitt entlang Linie XIV-XIV von Fig. 2 dar, wobei das Betätigungsteil 9 aus Veranschaulichungsgründen weggelassen ist, die Vorrichtung 1 bzw. das Mittelteil 5 weist beim Darstellungsbeispiel vorzugsweise einen festen Anschlag 5C auf, so dass das Innenteil 8 nur in einer Richtung, hier in Richtung B, von dem Anschlag 5C weg in der Vorrichtung 1 bzw. im Mittelteil 5 drehbar oder bewegbar ist. Insbesondere wirkt der Anschlag 5C mit dem Vorsprung 8B des Innenteils 8 derart zusammen, dass ein entgegengesetztes Bewegen bzw. Drehen blockiert ist.

In der in Fig. 14 gezeigten Drehstellung befindet sich das Innenteil 8 ausgehend vom vormontierten Zustand bis zur Beendigung des Öffnens. Insbesondere ist das Innenteil 8 in dieser Drehlage, beim Darstellungsbeispiel vorzugsweise durch eine entsprechende Rasterhebung 5D des Gehäuseteils 5, gehalten, so dass auch bei Zurückdrehen des Vorderteils 3 in Richtung B vor Erreichen des vollständig eingeschraubten Zustand 5 bzw. des vollständigen Öffnens lediglich das Vorderteil 3 wieder herausgeschraubt bzw. vom Innenteil 8 abgeschraubt wird, das Innenteil 8 aber nicht in Richtung B mitdreht. Vielmehr erfolgt dieses Mitdrehen in Richtung B erst nach entsprechender Verrastung bzw. Drehverbindung es Vorderteils 3 mit dem Innenteil 8, wie bereits anhand von Fig. 13 erläutert. Erst durch diese Rast- oder Drehverbindung kann eine ausreichend hohe Drehkraft auf das Innenteil 14 ausgeübt werden, so dass der Vorsprung 8B die Rasterhebung 5E, insbesondere durch entsprechende Materialverformung, überwinden und das Innenteil 8 in die Gegenrichtung bzw. in Richtung B mit dem ersten Bedienteil bzw. Vorderteil 3 mitgedreht wird.

Nach dem Öffnen erfolgt das genannte Mischen durch Bedienen des ersten Bedienelements bzw. Vorderteils 3 in einer anderen Bedienrichtung, insbesondere in entgegengesetzter Richtung, besonders bevorzugt durch Drehen in die Richtung B. Dieses Drehen ist vorzugsweise irreversibel. Beim Darstellungsbeispiel wird dies dadurch erreicht, dass Rastnasen 5E am Mitteilteil 5 radial derart nach innen vorspringen und Hinterschneidungen bzw. Anschläge bilden oder sägezahnartig ausgebildet sind, dass diese von dem Vorsprung 8B des Innenteils 8 durch elastische Materialverformung überwunden werden können, ein Rückdrehen entgegen der Richtung B jedoch verhindert bzw. blockiert wird. Jedoch sind auch andere konstruktive Lösungen möglich.

Zum Mischen erfolgt also eine andere bzw. unterschiedliche Bedienbewegung als zum Öffnen. Insbesondere wird hier das gleiche Bedienelement, nämlich das Vorderteil 3, zum Öffnen in die eine Bewegungsrichtung A und zum Mischen in die entgegengesetzte Bewegungsrichtung B bewegt, insbesondere gedreht.

Das Drehen des Innenteils 8 in Richtung B bewirkt das gewünschte Mischen dadurch, dass das Betätigungsteil 9 in die Karpule 2 bewegt und/oder der erste Kolben 2G verschoben wird. Nachfolgend wird erläutert, wie dies besonders bevorzugt beim Darstellungsbeispiel realisiert wird.

Das Betätigungsteil 9 ist vorzugsweise stempelartig ausgebildet und/oder axial verstellbar bzw. verfahrbar oder verschiebbar. Ein vorderer Bereich bzw. ein der Karpule 2 benachbarter Bereich des Betätigungsteils 9 durchgreift das Innenteil 8 bzw. eine beispielsweise in Fig. 14 gezeigte Durchbrechung 8C des Innenteils 8 und erstreckt sich in die Karpule 2, um den Kolben 2G je nach Bedarf zu verschieben bzw. die Karpule 2 zu drücken. Dieser Bereich des Betätigungsteils 9 steht mit dem Innenteil 8 drehfest, aber axial verschieblich in Eingriff. Vorzugsweise weist dieser Bereich daher eine nicht runde und/oder mit Vorsprüngen oder Einbuchtungen versehene Außenkontur auf, die in komplementären Ausbuchtungen, Vorsprüngen oder dergleichen der Durchbrechung 8C des Innenteils 8 drehfest geführt ist. Diese Durchbrechung 8C wird von dem genannten Bereich des Betätigungsteils 9 durchgriffen, wie beispielsweise aus den Fig. 1 und 11 ersichtlich.

Das Betätigungsteil 9 ist in einem anderen Bereich, insbesondere im Bereich seines der Karpule 2 abgewandten Endes mit mindestens einem radialen Vorsprung, einem Außengewinde 9A oder dergleichen versehen, der bzw. das mit einem Innengewinde 7A, insbesondere gebildet im oder am Hinterteil 7, derart in Eingriff steht, dass bei Drehen des Betätigungsteils 9 relativ zum Hinterteil 7, hier in Richtung B (durch das Innenteil 8) das Betätigungsteil 9 zu der Karpule 2 hin bzw. in die Karpule 2 axial hinein bewegt wird. Das Gegengewinde, hier das Innengewinde 7A am Hinterteil 7 wird vorzugsweise drehfest gehalten, insbesondere durch drehfestes Halten oder Blockieren des Hinterteils 7 an der Vorrichtung 1. Mit anderen Worten, beim Mischen bzw. Drehen in Richtung B schraubt sich das Betätigungsteil 9 nach vorne, wobei die Karpule 2 insbesondere zusammen mit dem Betätigungsteil 9, Innenteil 8 und/oder Vorderteil 3 entsprechend gedreht wird, so dass vorzugsweise keine relativen Verdrehungen oder Torsionskräfte auftreten.

Besonders bevorzugt ist ein doppelgängiger Gewindeeingriff zwischen dem Betätigungsteil 9 und der Vorrichtung 1 bzw. dem Hinterteil 7 vorgesehen.

Bei der Bedienbewegung in Richtung B bzw. beim Drehen in Richtung B bzw. beim Mischen wird das Betätigungsteil 9 in die Karpule 2 hineinbewegt bzw. der erste Kolben 2G verschoben, so dass die Flüssigkeit 2C aus der ersten Kammer 2B in die zweite Kammer 2D überführt wird, wobei hierbei anfänglich der zweite Kolben 2H insbesondere etwas nach unten bzw. zum Auslass hin verschoben wird, bis die Flüssigkeit 2C den Überströmkanal 2F erreicht und über diesen in die zweite Kammer 2D überströmen bzw. zu dem dort befindlichen Arzneimittel 2E strömen kann. Dieses Überströmen der Flüssigkeit 2C und Mischen erfolgt besonders bevorzugt bei etwas nach oben gerichtetem Auslass 4C, so dass in der Karpule 2 vorhandene Luft über den beim Öffnen geöffneten Verschluss 2J, beim Darstellungsbeispiel durch das Anstechelement 4A, den Auslass 4C und/oder den anliegenden Saugkörper 4E entweichen kann.

Fig. 15 zeigt den sich nach dem Mischen ergebenden Endzustand. Die Flüssigkeit 2C wurde in die zweite Kammer 2D überführt. Der erste Kolben 2G ist insbesondere bis zur Anlage am zweiten Kolben 2H verschoben worden. Ggf. kann der zweite Kolben 2H auch etwas weiter in die Karpule 2 hineingeschoben worden sein in diesem Zustand.

Nach dem Überführen bzw. Überströmen der Flüssigkeit 2C kann sich diese mit dem Arzneimittel 2E mischen, insbesondere das Arzneimittel 2E lösen. Dies kann ggf. selbsttätig oder durch Schütteln oder dergleichen erfolgen oder unterstützt werden.

Ggf. wird nach dem Überführen der Flüssigkeit 2C in die zweite Kammer 2D etwas zugewartet, bis das Lösen des Arzneimittels 2E erfolgt ist.

Das Mischen ist vorzugsweise visuell verfolgbar oder sichtbar. Insbesondere sind hierzu das Vorderteil 3 und Karpulengehäuse 2I zumindest teilweise oder insgesamt transparent ausgebildet.

Fig. 16 zeigt in einer ausschnittsweisen Vergrößerung, dass das Betätigungselement 9 am Ende der Bedienbewegung B bzw. des Mischens vorzugsweise noch nicht das axiale Ende des Innengewindes 7A erreicht hat.

Das Bewegen bzw. Drehen in die Richtung B, also die Bedienbewegung B, erfolgt vorzugsweise um weniger als 360°, beispielsweise etwa um 270° bis 320°. Insbesondere wird das Betätigungsteil 9 nicht vollständig in dem Innengewinde zur Karpule 2 hin axial vorwärtsbewegt bzw. vorwärtsgeschraubt. Vielmehr ist hier eine weitere axiale Bewegung des Betätigungsteils 9 durch relatives Verdrehen zu dem Innengewinde 7A bei der nächsten Bedienbewegung, nämlich beim Primen, noch möglich, worauf später näher eingegangen wird.

Das Hinterteil 7 bildet vorzugsweise ein (weiteres bzw. zweites) Bedienelement der Vorrichtung 1 und ist insbesondere zum Primen und Austragen bewegbar bzw. bedienbar, und zwar besonders bevorzugt in einer ersten Bedienbewegung oder -richtung C, besonders bevorzugt durch Drehen, und/oder in einer zweiten, anderen Bedienbewegung bzw. -richtung D, besonders bevorzugt in axialer Richtung.

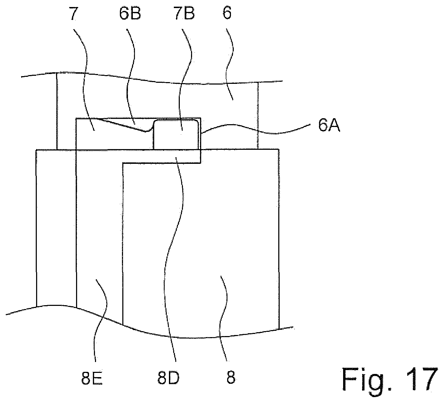

Während des Öffnens und/oder Mischens bzw. während der Bedienbewegungen A und/oder B ist das zweite Bedienelement bzw. Hinterteil 7 vorzugsweise gegen Bedienen bzw. Bewegen und/oder gegen Drehen bzw. Verdrehen und/oder axiales Bewegen blockiert bzw. gesperrt. Beim Darstellungsbeispiel erfolgt dies vorzugsweise über mindestens einen radialen Vorsprung 7B des Hinterteils 7, der in einer vorzugsweise axial offenen Ausnehmung 6A des Einsatzteils 6 drehfest gehalten ist, wie in der schematischen Darstellung gemäß Fig. 17 gezeigt. Insbesondere wird der Vorsprung 7B durch eine Halteerhebung 6B in der Ausnehmung 6A derart gehalten, dass bei axial glatt anliegender Stirnfläche des Innenteils 8 am Einsatzteil 6 der Vorsprung 7B die Halteerhebung 6B in Drehrichtung C nicht überwinden kann, sondern formschlüssig drehfest gehalten wird. Weiter bildet die glatte Stirnfläche des Innenteils 7 dann vorzugsweise einen axialen Anschlag für den Vorsprung 7B, so dass das Hinterteil 7 entsprechend axial gegen ein Eindrücken bzw. axiales Bewegen bzw. Bewegen in Richtung D gesichert oder blockiert ist.

Erst wenn nach dem Mischen die in Fig. 17 gezeigte Drehlage des Innenteils 8 relativ zum Einsatzteil 6 bzw. zur Ausnehmung 6A erreicht wird, kommt eine axiale Aussparung 8D des Innenteils 8 benachbart zur Ausnehmung 6A zu liegen, so dass nun zum Primen eine Bedienbewegung in Richtung C bzw. ein Drehen des Hinterteils 7 in Richtung C, insbesondere bei einem geringfügigen axialen Eindrücken, ermöglicht wird, da so der Vorsprung 7B an der Halteerhebung 6B vorbeibewegt werden kann, also umfangsmäßig in oder entlang der Ausnehmung 6A bewegt werden kann.

Die genannte Bedienbewegung C bzw. das genannte Drehen des Hinterteils 7 in Richtung C führt zu dem gewünschten Primen, indem das Innengewinde 7A relativ zum Betätigungsteil 9 bzw. dessen Außengewinde 9A (weiter) gedreht oder verdreht wird, wodurch das von dem Innenteil 8 drehfest geführte Betätigungsteil 9 (weiter) axial nach vorne bewegt bzw. geschoben und dadurch der erste Kolben 2G und entsprechend auch der zweite Kolben 2H weiter axial bewegt bzw. vorgeschoben oder vorgedrückt werden. Dementsprechend kann so in der zweiten Kammer 2D bzw. in der Karpule 2 und/oder im Auslass 4C hin eventuell vorhandene Luft durch die flüssige Arzneimittelformulierung 2A verdrängt bzw. ausgetrieben werden. Die dabei austretende Arzneimittelformulierung 2A wird vorzugsweise direkt von dem Saugkörper 4E aufgenommen bzw. aufgesaugt. Das Primen erfolgt also vorzugsweise bei aufgesetzter bzw. bei noch nicht abgenommener Abdeckung 4D.

Am Ende der Bedienbewegung in Richtung C erreicht das Innenteil 8 vorzugsweise seine zweite Endlage in Drehrichtung B. Insbesondere wird beim Darstellungsbeispiel der Vorsprung 8B zwischen dem Vorsprung 5B und der nächsten benachbarten Rastnase 5E gehalten, insbesondere also auf der in Fig. 14 rechten Seite des Vorsprungs 5B.

Fig. 18 zeigt in einem schematischen Schnitt den Zustand nach dem Primen. Das Betätigungsteil 9 und die Kolben 2G und 2H wurden etwas mehr axial vorgeschoben bzw. zum Auslass 4C hin bewegt. Jetzt kann die Abdeckung 4D der Vorrichtung 1 entfernt bzw. geöffnet werden und das eigentliche Austragen bzw. Ausgeben der flüssigen Arzneimittelformulierung 2A über den Auslass 4C erfolgen. Dies erfolgt besonders bevorzugt durch eine weitere (unterschiedliche) Bedienbewegung D. Diese verläuft insbesondere in einer anderen Richtung als die Bedienbewegung C zum Primen. Diese erfolgt vorzugsweise wieder mit dem gleichen Bedienelement, hier über das Hinterteil 7. Besonders bevorzugt erfolgt eine axiale oder lineare Bewegung, insbesondere ein lineares oder axiales Eindrücken des Hinterteils 7 in die Vorrichtung 1 bzw. das Mittelteil 5, wie durch Pfeil D insbesondere in Fig. 1 angedeutet.

Um die vorgenannte axiale Bewegung bzw. Bewegung des Hinterteils 7 in Richtung D und damit des Betätigungsteils 9 und der Kolben 2G und 2H in Richtung D zu ermöglichen, muss das Hinterteil 7 bzw. dessen Vorsprung 7B in axialer Richtung D freigegeben werden. Dies erfolgt beim Darstellungsbeispiel vorzugsweise dadurch, dass nach dem Primen durch Bewegen bzw. Drehen in Richtung C der Vorsprung 7B axial benachbart bzw. in Verlängerung einer Axialnut 8E des Innenteils 8 zu liegen kommt (die Axialnut 8E ist in Fig. 17 angedeutet), so dass dann die Axialbewegung in Richtung D freigegeben wird.

Beim Darstellungsbeispiel sind vorzugsweise an gegenüberliegenden Seiten Vorsprünge 7B gebildet. Dementsprechend sind dann auch jeweils zwei Ausnehmungen 6A, Haltererhebungen 6B, Aussparungen 8D und/oder Axialnuten 8E gebildet.

Jedoch sind grundsätzlich auch andere konstruktive Lösungen möglich, um die gewünschte Sperrung und Freigabe zu realisieren.

Durch die Bedienbewegung D bzw. das axiale Eindrücken des (zweiten) Bedienelements bzw. Hinterteils 7 wird die flüssige Arzneimittelformulierung 2A aus der Vorrichtung 1 bzw. Karpule 2 in gewünschter Weise ausgetragen bzw. ausgegeben, hier besonders bevorzugt über den Auslass 4C bzw. einen davon gebildeten Anschluss. Alternativ oder zusätzlich kann die Ausgabe auch über eine Kanüle, beispielsweise zur direkten Injektion oder dergleichen erfolgen.

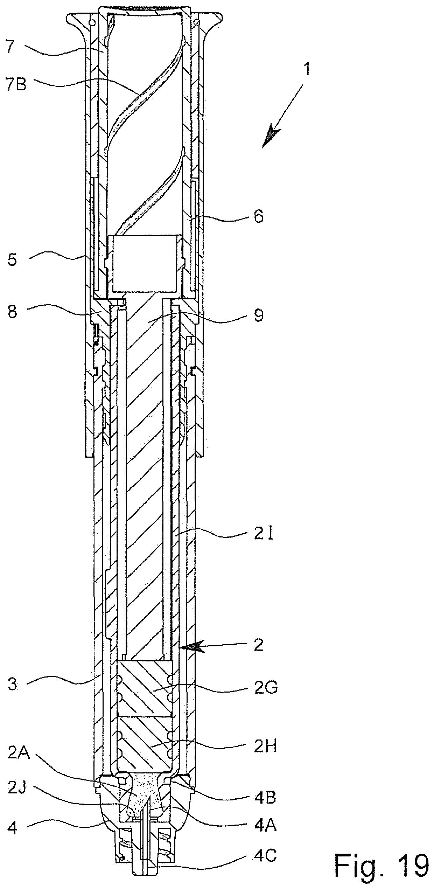

Nach dem Austragen bzw. bei Erreichen der Endposition der Bedienbewegung D ist das zweite Bedienelement bzw. Hinterteil 7 vorzugsweise zumindest im Wesentlichen vollständig in das Mittelteil 5 bzw. Gehäuse der Vorrichtung 1 eingedrückt oder darin aufgenommen, insbesondere so, dass eine weitere Bedienung nicht mehr möglich ist, wie aus der schematischen Darstellung gemäß Fig. 19 ersichtlich, die den Zustand nach dem Austragen veranschaulicht. Jedoch ist es auch unproblematisch, wenn das Hinterteil 7 und das Betätigungsteil 9 wieder zurück gezogen werden, da hierdurch der Kolben 2G nicht zurückbewegt oder -gezogen wird, also kein Ansaugen erfolgt.

Besonders bevorzugt bilden das Betätigungsteil 9 und das Hinterteil 7 einen Stößel der Vorrichtung 1, durch den eine Betätigung der Karpule 2 bzw. eine Verschiebung des (ersten) Kolbens 2G bzw. der Kolben 2G, 2H der Karpule 2 ermöglicht wird oder erfolgt. Dieser Stößel ist beim Darstellungsbeispiel vorzugsweise mehrteilig ausgebildet, hier nämlich durch die Teile 7 und 9, und durch Verdrehen (insbesondere durch relatives Verdrehen der beiden Teile 7 und 9 zueinander) verlängerbar. Dies gestattet eine gute bzw. einfache Handhabung. Insbesondere wird bei kompakten Aufbau der Vorrichtung 2 ermöglicht, dass ein nicht dargestellter Benutzer nicht umgreifen muss und/oder immer noch eine ausreichende Angriffsfläche hat.

Besonders bevorzugt ist die vorschlagsgemäße Vorrichtung 1 nämlich derart bedienbar, dass die erste Baueinheit bzw. das erste Bedienelement, wie das Gehäuseteil 3, mit einer Hand und die zweite Baueinheit bzw. Oberteil oder Mittelteil 5 mit der anderen Hand erfasst werden kann, wobei eine Bedienung des (zweiten) Bedienelements bzw. Hinterteils 7 mittels des Daumens oder eines andren Fingers der anderen Hand ohne Umgreifen erfolgen kann. Dementsprechend wird eine sehr einfache und sichere Handhabung ermöglicht.

Die vorschlagsgemäße Vorrichtung 1 und ein vorschlagsgemäßes Verfahren zur Betätigung einer Karpule zeichnen sich insbesondere auch dadurch aus, dass in ihrer Richtung unterschiedliche Bedienbewegungen oder Bedienbewegungen in entgegengesetzte Richtungen nacheinander ausgeführt werden und/oder unterschiedliche Bedienelemente, wie das Vorderteil 3 einerseits und das Hinterteil 7 andererseits, nach einander bedient werden, insbesondere um unterschiedliche oder verschiedene Vorgänge bzw. Schritte, wie das Öffnen, Mischen, Primen und/oder Austragen, zu bewirken.

Ein weiterer Aspekt der vorliegenden Erfindung liegt insbesondere darin, dass bei der vorschlagsgemäßen Vorrichtung 1 bzw. bei dem vorschlagsgemäßen Verfahren einige oder alle Bedienbewegungen und/oder einige oder alle Vorgänge bzw. Schritte, wie Öffnen, Mischen, Primen und/oder Austragen, nacheinander und/oder definiert ausgeführt oder bewirkt werden können.

Besonders bevorzugt ist für jeden Vorgang bzw. Schritt, wie Öffnen, Mischen, Primen und/oder Austragen, also für unterschiedliche Betätigungen der Karpule 2, jeweils eine andere oder unterschiedliche Bedienbewegung bzw. eine andere Bedienrichtung und/oder ein anderes Bedienelement erforderlich. Dies erleichtert eine einfache, intuitive und/oder sichere Bedienung bzw. Handhabung der Vorrichtung 1.

Die vorschlagsgemäße Vorrichtung 1 ist relativ einfach, insbesondere aus Kunststoffteilen bzw. Spritzgussteilen und/oder aus wenigen Teilen aufgebaut.

Besonders bevorzugt ist die Vorrichtung 1 mit entsprechenden Kennzeichnungen oder Symbolen, wie den in Fig. 1 dargestellten Pfeilen oder Piktogrammen, zur Veranschaulichung der Bedienbewegungen bzw. erforderlichen Bedienungen versehen. Die verschiedenen Kennzeichnungen sind insbesondere nummeriert oder in sonstiger Weise markiert, um einen nicht dargestellten Benutzer die gewünschte oder erforderliche Reihenfolge zu zeigen oder zu veranschaulichen.

Weiter ist es möglich, dass einzelne Kennzeichnungen nach erfolgter Bedienung bzw. Ausführung der entsprechenden Bedienbewegung nicht mehr sichtbar sind. Beispielsweise kann die erste Kennzeichnung mit dem Pfeil A nach dem Einschrauben des Vorderteils 3 im Mittelteil 5 verschwinden bzw. von diesem verdeckt werden. Ein Benutzer wird dann automatisch bzw. intuitiv mit der nächsten Bedienung bzw. Bedienbewegung, hier in Richtung B, fortfahren.

Die vorschlagsgemäße Vorrichtung 1 und das vorschlagsgemäße Verfahren sind grundsätzlich und gemäß einer bevorzugten Variante auch für sonstige Zwecke, insbesondere zum Mischen von Stoffen und/oder Flüssigkeiten generell und zum Ausgeben, bspw. für Mehr- oder Zweikomponentensysteme oder -mischungen oder -gemische, wie Zweikomponentenklebstoffe, einsetzbar. Die Karpule 2 enthält dann dementsprechend andere Stoffe und/oder Flüssigkeiten. Ggf. kann dann auch das Primen entfallen.

### Bezugszeichenliste:

- 1: Vorrichtung
- 2: Karpule
- 2A: Arzneimittelformulierung
- 2B: erste Kammer
- 2C: Flüssigkeit
- 2D: zweite Kammer
- 2E: Arzneimittel
- 2F: Überströmkanal
- 2G: erster Kolben
- 2H: zweiter Kolben
- 2I: Gehäuse
- 2J: Verschluss
- 3: Vorderteil
- 3A: Außengewinde
- 3B: Innengewinde
- 3C: Rastausnehmung
- 3D: Vorsprung
- 3E: Freigang
- 4: Kopfteil
- 4A: Anstechelement
- 4B: Ringabschnitt
- 4C: Auslass
- 4D: Abdeckung
- 4E: Saugkörper
- 5: Mittelteil
- 5A: Innengewinde
- 5B: Vorsprung
- 5C: Anschlag
- 5D: Rasterhebung
- 5E: Rastnase
- 5F: Ausnehmung
- 6: Einsatzteil
- 6A: Ausnehmung
- 6B: Halteerhebung
- 6C: Vorsprung
- 7: Hinterteil
- 7A: Innengewinde
- 7B: Vorsprung
- 8: Innenteil
- 8A: Außengewinde
- 8B: Vorsprung
- 8C: Durchbrechung
- 8D: Aussparung
- 8E: Axialnut
- 9: Betätigungsteil
- 9A: Außengewinde
- A: Bedienbewegung bzw. -richtung
- B: Bedienbewegung bzw. -richtung
- C: Bedienbewegung bzw. -richtung
- D: Bedienbewegung bzw. -richtung

## Patentansprüche

1. Vorrichtung (1) zur Aufnahme und Betätigung einer Karpule (2) mit einem Kolben (2G), um eine flüssige Arzneimittelformulierung (2A) oder sonstige Flüssigkeit aus der Karpule (2) auszugeben, wobei die Vorrichtung (1) einen zur Verschiebung des Kolbens (2G) axial verschiebbaren Stößel aufweist, wobei
der Stößel mehrteilig ausgebildet und durch Verdrehen teleskopisch verlängerbar ist,
die Vorrichtung (1) derart ausgebildet ist, dass mindestens eine Drehbewegung und eine Axialbewegung als Bedienbewegung kombiniert werden, und
die Vorrichtung (1) ein nacheinander in entgegengesetzte Richtungen drehbares erstes Bedienelement zur Bedienung der Vorrichtung (1) und ein Innenteil (8) aufweist, und
das erste Bedienelement der Vorrichtung (1) zum Öffnen der Karpule (2) in eine erste Richtung (A) drehbar ist und das Bedienelement einen axialen Hinterschnitt, Anschlag oder Vorsprung (3D) aufweist, der nach Vorbeibewegen an einem radialen Vorsprung (8B) des Innenteils (8) zur Anlage kommt, mittels dessen das Bedienelement bei Abschluss der ersten Drehung mit dem Innenteil (8) verrasten und drehfest verkoppelt werden kann, und
**dadurch gekennzeichnet,**
**dass** das erste Bedienelement mit dem Innenteil (8) drehfest gekoppelt in eine entgegengesetzte Richtung (B) drehbar ist, wobei das Drehen in die entgegengesetzte Richtung (B) zum Mischen einer Flüssigkeit (2C) mit einem trockenen Arzneimittel (2E) oder zum Mischen von zwei Flüssigkeiten (2A, 2C) in der Karpule (2) die Bewegung eines Betätigungsteils (9) und/oder die Verschiebung eines Kolbens (2G) bewirkt.

2. Vorrichtung nach Anspruch 1, **dadurch gekennzeichnet, dass** die Vorrichtung (1) neben dem ersten Bedienelement zum Öffnen der Karpule (2) und/oder Mischen einer Flüssigkeit (2C) mit einem trockenen Arzneimittel (2E) in der Karpule (2) ein zweites, separates Bedienelement zum Primen der Karpule (2) und/oder zum Austragen der flüssigen Arzneimittelformulierung (2A) aus der Karpule (2) aufweist.

3. Vorrichtung nach Anspruch 2, **dadurch gekennzeichnet, dass** das zweite Bedienelement zum Primen der Karpule (2) drehbar und zum Austragen der flüssigen Arzneimittelformulierung (2A) axial eindrückbar ist.

4. Vorrichtung nach einem der voranstehenden Ansprüche, **dadurch gekennzeichnet, dass** einzelne oder alle Bedienbewegungen gegen ein Bewegen in die entgegengesetzte Richtung gesperrt sind.

5. Vorrichtung nach einem der voranstehenden Ansprüche, **dadurch gekennzeichnet, dass** die Vorrichtung (1) derart ausgebildet ist, dass nacheinander ein Öffnen, Mischen, Primen und Austragen durch unterschiedliche Bedienbewegungen (A bis D) bewirkt werden.

6. Vorrichtung nach Anspruch 2 oder 3, **dadurch gekennzeichnet, dass** der Stößel aus dem Betätigungselement (9) und dem zweiten Bedienelement bzw. Hinterteil (7) gebildet ist, wobei das Betätigungsteil (9) mit einem Ende in die Karpule (2) einführbar ist und mit dem anderen Ende in das andere Teil, insbesondere über einen Gewindeeingriff, eingreift.

7. Vorrichtung nach einem der voranstehenden Ansprüche, **dadurch gekennzeichnet, dass** die Vorrichtung (1) zwei vorzugsweise hülsenartig oder hohlzylindrisch ausgebildete, zueinander verdrehbare Teile (3, 5) aufweist, die insbesondere ein zumindest im Wesentlichen zylindrisches Gehäuse der Vorrichtung (1) bilden.

8. Vorrichtung nach einem der voranstehenden Ansprüche, **dadurch gekennzeichnet, dass** die Karpule (2) als Mehrkammerkarpule ausgeführt ist.

## Claims

1. Device (1) for receiving and actuating a carpule (2) with a piston (2G), in order to expel
a liquid medicament formulation (2A) or other liquid from the carpule (2), the device (1)
comprising a ram that is axially movable for displacing the piston (2G),
wherein the ram is of multi-part configuration and is telescopically extensible by rotation,
the device (1) is embodied such that at least one rotary movement and one axial movement are combined as an operating movement, and
the device (1) comprises a first operating element for operating the device (1) that is rotatable in opposite directions one after the other and an inner part (8), and
the first operating element of the device (1) is rotatable in a first direction (A) for opening the carpule (2) and the operating element comprises an axial undercut, abutment or projection (3D) which after moving past, comes to abut on a radial projection (8B) of the inner part (8) and by which the operating element can latch and couple for rotation with the inner part (8) when the first rotation has ended, and
**characterised in**
**that** when rotary coupled to the inner part (8), the first operating element is rotatable in an opposite direction (B), wherein the rotation in the opposite direction (B) causes the movement of an actuating part (9) and/or the displacement of a piston (2G) for mixing a liquid (2C) with a dry medicament (2E) or for mixing two liquids (2A, 2C) in the carpule (2).

2. Device according to claim 1, **characterised in that** the device (1) comprises, in addition to the first operating element for opening the carpule (2) and/or mixing a liquid (2C) with a dry medicament (2E) in the carpule (2), a second, separate operating element for priming the carpule (2) and/or for expelling the liquid medicament formulation (2A) from the carpule (2).

3. Device according to claim 2, **characterised in that** the second operating element is rotatable for priming the carpule (2) and can be pressed in axially for expelling the liquid medicament formulation (2A).

4. Device according to one of the preceding claims, **characterised in that** individual or all operating movements are blocked against movement in the opposite direction.

5. Device according to one of the preceding claims, **characterised in that** the device (1) is configured such that opening, mixing, priming and expulsion are effected successively by different operating movements (A to D).

6. Device according to claim 2 or 3, **characterised in that** the ram is formed from the actuating element (9) and the second operating element or rear part (7), the actuating part (9) being insertable with one end into the carpule (2) and engaging with the other end in the other part, particularly by a screw-threaded engagement.

7. Device according to one of the preceding claims, **characterised in that** the device (1) comprises two parts (3, 5) that are rotatable relative to one another and are preferably of sleeve-shaped or hollow-cylindrical configuration, which form in particular an at least substantially cylindrical housing of the device (1).

8. Device according to one of the preceding claims, **characterised in that** the carpule (2) is embodied as a multi-chamber carpule.

## Revendications

1. Dispositif (1) pour la réception et l'actionnement d'une carpule (2) avec un piston (2G), afin de délivrer une formulation médicamenteuse (2A) liquide ou autre liquide depuis la carpule (2), dans lequel le dispositif (1) présente un coulisseau mobile axialement pour le déplacement du piston (2G), dans lequel
le coulisseau est réalisé en plusieurs parties et est prolongeable de manière télescopique par rotation,
le dispositif (1) est réalisé de telle manière qu'au moins un mouvement rotatif et un mouvement axial soient combinés comme mouvement de commande, et
le dispositif (1) présente un premier élément de commande pouvant tourner l'un après l'autre dans des sens opposés pour la commande du dispositif (1) et une partie intérieure (8), et
le premier élément de commande du dispositif (1) est rotatif pour l'ouverture de la carpule (2) dans un premier sens (A) et l'élément de commande présente une contre-dépouille, butée ou saillie axiale (3D) qui vient en appui après le passage devant une saillie (8B) radiale de la partie intérieure (8), au moyen de laquelle l'élément de commande peut s'encliqueter et être couplé sans pouvoir tourner lors de la fin de la première rotation avec la partie intérieure (8), et **caractérisé en ce**
**que** le premier élément de commande est rotatif dans un sens opposé (B) de manière couplée sans pouvoir tourner avec la partie intérieure (8), dans lequel la rotation dans le sens opposé (B) provoque pour le mélange d'un liquide (2C) avec un médicament (2E) sec ou pour le mélange de deux liquides (2A, 2C) dans la carpule (2) le mouvement d'une partie d'actionnement (9) et/ou le déplacement d'un piston (2G).

2. Dispositif selon la revendication 1, **caractérisé en ce que** le dispositif (1) présente, outre le premier élément de commande pour l'ouverture de la carpule (2) et/ou le mélange d'un liquide (2C) avec un médicament (2E) sec dans la carpule (2), un second élément de commande séparé pour amorcer la carpule (2) et/ou pour évacuer la formulation médicamenteuse (2A) liquide de la carpule (2).

3. Dispositif selon la revendication 2, **caractérisé en ce que** le second élément de commande est rotatif pour amorcer la carpule (2) et enfonçable axialement pour évacuer la formulation médicamenteuse (2A) liquide.

4. Dispositif selon l'une des revendications précédentes, **caractérisé en ce que** des mouvements de commande individuels ou tous sont bloqués contre un déplacement dans le sens opposé.

5. Dispositif selon l'une des revendications précédentes, **caractérisé en ce que** le dispositif (1) est réalisé de telle manière qu'une ouverture, un mélange, un amorçage et une évacuation soient provoqués les uns après les autres par des mouvements de commande (A à D) différents.

6. Dispositif selon la revendication 2 ou 3, **caractérisé en ce que** le coulisseau est formé à partir de l'élément d'actionnement (9) et le second élément de commande ou partie arrière (7), dans lequel la partie d'actionnement (9) peut être introduite avec une extrémité dans la carpule (2) et vient en prise avec l'autre extrémité dans l'autre partie, en particulier par le biais d'une prise filetée.

7. Dispositif selon l'une des revendications précédentes, **caractérisé en ce que** le dispositif (1) présente deux parties (3, 5) rotatives l'une par rapport à l'autre, réalisées de préférence comme une douille ou un cylindre creux qui forment en particulier un boîtier au moins sensiblement cylindrique du dispositif (1).

8. Dispositif selon l'une des revendications précédentes, **caractérisé en ce que** la carpule (2) est réalisée comme carpule à plusieurs chambres.
